# EUROPEAN PATENT APPLICATION

(11) **EP 4 365 192 A1**
(43) Date of publication of application: **08.05.2024**
(21) Application number: 22205462.9
(22) Date of filing: 04.11.2022
(51) Int. Cl.: C07K 14/50

(54) **MICROBIAL PRODUCTION OF GROWTH FACTORS**

(71) Applicant: DSM IP Assets B.V., 6411 TE Heerlen (NL)
(72) Inventor: Van Rij, Evert Tjeerd, 6100 AA Echt (NL); Van Leeuwen, Johannes Gustaaf Ernst, 6100 AA Echt (NL)
(74) Representative: dsm-firmenich IP

(57) **Abstract**

The present invention relates to an isolated polypeptide, or a functional fragment thereof, having at least 85% sequence identity to SEQ ID NO: 12, comprising an amino acid substitution at a position selected from the group consisting of: C12, Q43, A62, H89 and C113.

## Description

### Field

The invention relates to the recombinant production of polypeptides, specifically to the field of microbial production of growth factors.

### Background

Cultivated meat, meat production through cell culture in bioreactors is a hot topic and many start-up companies are active. A challenge is to improve and upscale the cell culture fermentation process and the production of less expensive food grade growth factors. Growth factors are the most expensive medium ingredient and are essential to keep cells proliferating.

Mature (pig) FGF2 is a 146 amino acid polypeptide of 16 kDa. It's involved in embryonic development, cell growth, morphogenesis, tissue repair, tumour growth and invasion. FGF2 is required to support proliferation and inhibit differentiation. Stability of FGF2 in aqueous solutions is a major concern in the development of medical products (Benington, 2020). Sigma offers a 16 kDa human FGF2 produced by *E. coli* at a price of 1030 EURO for 100 µgram (10.300.000 EURO/gram). This is lipolyzed and stabilized with bovine serum albumin (BSA). The mature protein sequences of the human, pig and bovine differ only at three AA positions. Although FGF2 contains four cytidines they do not form disulphide brides, which make the protein sensitive to oxidation and aggregation. FGF2 can be stabilized with BSA and heparin (Benington, 2020). However, stabilizers from animal origin have drawbacks since the whole idea behind cultivated meat is reduce the use animal resources. Further, batch to batch variation of animal heparin and possible contamination like viruses is a problem. In addition, BSA and heparin may contain pathogens. Production of FGF2 is described in literature for *E. coli* and *B. subtilis* (Seddon, 1991, Seeger, 1995, Soleyman, 2016, US2019338007 AA). *E. coli* is less attractive for the producing food grade proteins, as extensive purification is needed to remove all endotoxin and this host is not accepted in some food grade production plants.

There is thus a need to improve the stability of growth factors, reduce the price to produce them and to find an alternative microbial host to produce growth factors.

### Detailed description

The present invention relates to an isolated polypeptide, or a functional fragment thereof, having at least 85% sequence identity to SEQ ID NO: 12, comprising an amino acid substitution at a position selected from the group consisting of: C12, Q43, A62, H89 and C113.

Surprisingly, the present inventors found that the indicated amino acid substitutions provide an increased stability to the polypeptide, which can beneficially be used as a growth factor.

Preferably, the present polypeptide is a FGFc molecule. FGFc means that the polypeptide is a chimera, preferably a chimera of FGF1 and FGF2.

Preferably, the present polypeptide remains active even after 24 or 48 hours incubation at 37°C. Preferably, the present polypeptide maintains at least 50%, at least 60%, at least 70%, at least 80%, at least 90% or at least 95% of its activity after 24 hours or 48 hours incubation at 37°C. Preferably the activity is measured by the absorbance at 450 nm in an ELISA assay, preferably the assay as used in the present examples. Preferably the present polypeptide has an absorbance of at least 2 measured at 450 nm.

In other words, the present polypeptide has preferably a residual activity of at least 50%, at least 60%, at least 70%, at least 80%, at least 90%, at least 95 or at least 99% of its activity at t=0, after 24 hours or 48 hours incubation at 37°C, preferably in a standard buffer.

Alternatively, the present polypeptide has an EC50 value in a (HEK293) activity assay, of less than a tenfold relative increase after 24 or 48 hours incubation at 37°C relative to t=0. Preferably the EC50 value is less than a fivefold increase after 24 hours incubation at 37°C relative to t=0. Preferably, the present polypeptide has an EC50 value in a (HEK293) activity assay, within the range of 1 to 20 times the EC50 value at t=0.

Preferably, the sequence identity to SEQ ID NO: 12 is at least 86%, 87%, 88%, 89%, 90%, 91%, 92%, 93%, 94%, 95%, 96%, 97%, 98% or at least 99%.

Preferably, the present polypeptide has an increased stability when compared to a polypeptide having a similar sequence identity, but without an amino acid substitution at a position selected from the group consisting of: C12, Q43, A62, H89 and C113. Preferably the present polypeptide has an increased stability when compared to a polypeptide having a similar sequence identity, but without the amino acid substitution C12, Q43, A62, H89 and C113.

Preferably, the present polypeptide comprises two, three, four or even five amino acid substitution at a position selected from the group consisting of: C12, Q43, A62, H89 and C113.

Preferably, the present polypeptide comprises an amino acid substitution at a position: C12 and C113 or Q43, A62 and H89.

Preferably, the present amino acid substitution at a position C12 and C113 are chosen from C12S, C12A, C12T, C113S, C113A and C113T.

In a preferred embodiment, the amino acid substitutions are selected from the group consisting of C12S, Q43I, A62C, H89G and C113A.

Preferably, the present polypeptide further comprises an amino acid substitution at position K108, preferably K108T.

Preferably, the present polypeptide comprises the following amino acid substitutions:
C12S, Q43I, A62C and H89G
C12S, Q43I, A62C and C113A
C12S, Q43I, H89G and C113A
C12S, A62C, H89G and C113A; or
Q43I, A62C, H89G and C113A.

In a preferred embodiment, the present isolated polypeptide, or a functional fragment thereof, comprises or consists of a polypeptide with the sequence as set forward in SEQ ID NO's 13 to 15. Preferably as set forward in SEQ ID NO 15.

According to another aspect, the present invention relates to a polynucleotide encoding a polypeptide as described herein. Preferably, the polynucleotide comprises or consists of a sequence as set forward in SEQ ID NO's 18 to 20.

According to another aspect, the present invention relates to an expression vector comprising the polynucleotide as described herein.

According to another aspect, the present invention relates to a host cell comprising the expression vector as described herein. Preferably the host cell is a yeast. Preferably a yeast from the genus *Saccharomyces, Kluyveromyces* or *Pichia.* Preferably the host cell is a *Pichia Pastoris* or *Komagataella phaffii.*

According to another aspect, the present invention relates to a method for the production of the present polypeptide, comprising culturing the host cell as described herein under conditions conducive to the production of the polypeptide, and optionally, isolating and/or purifying the polypeptide from the broth. Preferably, the present method comprises a step of lysis of host cells using octanol.

According to another aspect, the present invention relates to a culture medium comprising the present polypeptide. Preferably, the culture medium comprises an amount of polypeptide within the range of 0.5-100 ng/ml, more preferably 1 to 50 ng/ml, most preferably around 10ng/ml. Preferably, the culture medium is suitable to be used for the growth of cells, wherein the cells preferably are non-human mammalian cells, such as pig cells. Preferably the cells are chosen from the group of pig cells, bovine cells, sheep cells, goat cells, chicken cells, duck cells, fish cells, salmon cells, trout cells and tuna cells.

According to another aspect, the present invention relates to a method for culturing cells comprising incubating cells in a culture medium comprising the present polypeptide, wherein the cells preferably are non-human mammalian cells. Preferably wherein the cells are pig cells. Preferably the cells are chosen from the group of pig cells, bovine cells, sheep cells, goat cells, chicken cells, duck cells, fish cells, salmon cells, trout cells and tuna cells.

According to another aspect, the present invention relates to a cultured cell obtainable by the method for culturing cells comprising incubating cells in a culture medium comprising the present polypeptide, wherein the cells preferably are non-human mammalian cells. Preferably wherein the cells are pig cells. Preferably, the cultured cell is a pig cell. Preferably the cells are chosen from the group of pig cells, bovine cells, sheep cells, goat cells, chicken cells, duck cells, fish cells, salmon cells, trout cells and tuna cells.

According to another aspect, the present invention relates to method for the production of a food product comprising culturing non-human mammalian cells in a culture medium comprising the present polypeptide, and optionally, isolating and/or purifying the food product from the broth. Preferably wherein the non-human mammalian cells are pig cells. Preferably the cells are chosen from the group of pig cells, bovine cells, sheep cells, goat cells, chicken cells, duck cells, fish cells, salmon cells, trout cells and tuna cells.

According to another aspect, the present invention relates to a food product obtainable by to method for the production of a food product comprising culturing non-human mammalian cells in a culture medium comprising the present polypeptide, and optionally, isolating and/or purifying the food product from the broth. Preferably wherein the food product is pork meat. Preferably the food product like a sausage. Preferably the cells are chosen from the group of pig cells, bovine cells, sheep cells, goat cells, chicken cells, duck cells, fish cells, salmon cells, trout cells and tuna cells.

Alternatively, the present invention relates to the use of the present polypeptide for the production of a vaccin, preferably a vaccin produced in a human or non human cell line.

The term "Sequence identity" is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences. "Similarity" between two amino acid sequences is determined by comparing the amino acid sequence and its conserved amino acid substitutes of one peptide or polypeptide to the sequence of a second peptide or polypeptide. In a preferred embodiment, identity or similarity is calculated over the whole SEQ ID NO as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A "nucleic acid molecule" or "polynucleotide" (the terms are used interchangeably herein) is represented by a nucleotide sequence. A "polypeptide" is represented by an amino acid sequence. A "polypeptide" as used herein refers to any peptide, oligopeptide, polypeptide, gene product, expression product, or protein. A polypeptide is comprised of consecutive amino acids. The term "polypeptide" encompasses naturally occurring and synthetic molecules.

The sequence information as provided herein should not be so narrowly construed as to require inclusion of erroneously identified bases. The skilled person is capable of identifying such erroneously identified bases and knows how to correct for such errors.

The invention is further illustrated in the non-limiting example below.

### Examples

Standard genetic techniques, such as overexpression of enzymes in the host cells, genetic modification of host cells, or hybridisation techniques, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Transformation procedure was performed according to condensed electroporation protocol using freshly prepared solutions (Lin-Cereghino J1, Wong WW, Xiong S, Giang W, Luong LT, Vu J, Johnson SD, Lin-Cereghino GP.Biotechniques. (2005) 38, (1):44-48).

### Materials and Methods

### Strains

*Pichia Pastoris* or *Komagataella phaffii* strain PPS-9016 was obtained from ATUM (formerly DNA2.0, Newark, CA, USA) and is used as expression host. Strain PPS-9016 has the following genotype: Mut+ Δpep4 and Δprb1.

### Plasmid pCASPP-01 containing CAS9 and guide RNA targeting LIG4

The functional deletion of LIG4 was accomplished using CRISPR-CAS9 (DiCarlo et al., 2013, Nucleic Acids Res 41:4336-4343) and WO16110512 and US2019309268. A so called "all in one" plasmid named pCASPP-Lig4 was constructed which expressed both CAS9 and the guide RNA (gRNA) from a pRS414 plasmid with CEN6 ARS4 Yeast centromere (Sikorski and Hieter, 1989; available from Stratagene GenBank:U03448). The TRP1 selection marker was replaced by a kanMX marker under control of *S. cerevisiae* TEF1 promoter and TEF1 terminator which confers resistance to the yeast cells against the antibiotic substance geneticin (G418) (SEQ ID 01). The *Streptococcus pyogenes* CAS9 gene expressed with the *S. cerevisiae* GAP1 promoter and CYC1 terminator (SEQ ID 02) and the LIG4 gRNA expressed with the pSER promoter (SEQ ID 03). The guide RNA and protospacer sequences were designed with a gRNA designer tool (https://www.atum.bio/eCommerce/cas9/input).

The pCASPP backbone was PCR amplified from pCASPP-01 using primers DBC-26957 (SEQ ID 04) and DBC-26958 (SEQ ID 05) using Phusion polymerase. The gRNA's were PCR amplified from the ordered gBlocks using DBC-26287 (SEQ ID 06) and DBC-26288 (SEQ ID 07) using Phusion polymerase. A Gibson reaction was performed to assembly the Lig4-1 gRNA into the pCASPP backbone to yield a functional AIO plasmid. *Pichia Pastoris* or *Komagataella phaffii* strain PPS-9016 was transformed with pCASPP-Lig4 and the Lig4 repair DNA (SEQ ID 08). Competent PPS02 was transformed with 100 ng plasmid pCASPP-Lig4 and 1000 ng of repair DNA. Plating was done on 48 well Q-trays containing Selective G418 (750 ug/ml) yepDS agar. Ten times and a fifty times dilution were plated and incubation at 30 °C for 3 days.

Colonies were checked by diagnostic PCR, with primers
DBC-29752 (SEQ ID 09).
DBC-29753 (SEQ ID 10).

Correct Lig4 deletion mutants were named PPS02 with the following genotype; Mut+, Δpep4, Δ prb1, Δlig4.

### Example 1: Cloning and expression of growth factors

### Cloning and Expression

The growth factors were expressed and produced using *Pichia pastoris* (currently renamed as *Komagataella phaffii*) host cells strain PPS02. DNA constructs encoding the polypeptides FGF301 (SEQ ID NO:11), FGF320 (SEQ ID NO:12), FGF321 (SEQ ID NO:13), FGF322 (SEQ ID NO:14), FGF323 (SEQ ID NO:15) were codon optimized for *Pichia pastoris* as described in WO2008/000632, resulting in DNA sequences SEQ ID NO: 16-20. *Bsa*I type II restriction enzyme sites were added to both sides of the DNA constructs (CGGTCTCGA-ORF-AGGAGACCG) as detailed in WO2013/144257A1 and WO2015/028582. The promoter, ORF and terminator sequences are assembled into expression cassettes with Golden Gate technology, as described by Engler et al (2011) and ligated into *Bsa*I-digested backbone vectors that decorated the expression cassettes with the connectors.

The synthetic DNA constructs were ordered at TWIST (South San Francisco, CA 94080, USA). Expression cassettes were compiled using Golden Gate Cloning and comprised a truncated *Hansenula polymorpha*/ *Ogataea polymorpha* FMD promoter, Opol_DF.pro_0001 (illustrated by SEQ ID NO:21) as described in (WO21250109 A1 - TRUNCATED PROMOTER FOR RECOMBINANT GENE EXPRESSION), the gene of interest coding and the *Pichia Pastoris* AOX1 terminator, Pp_AOX1.ter (illustrated by SEQ ID NO:22).

For each gene two expression cassettes were generated by PCR, one with the connectors A (SEQ ID NO:23) and B (SEQ ID NO:24) and a second one with connectors B (SEQ ID NO:24) and C (SEQ ID NO:25) to allow integration of two copies at the pTEFUP locus. The A-B cassettes obtained a 100 bp 5'-flank targeting the pTEFUP locus and were amplified with primers
DBC-29906: (SEQ ID NO:26)
DBC-05796: (SEQ ID NO:27)

The B-C cassettes obtained a 100 bp 3'-flank targeting the pTEFUP locus and were amplified with primers
DBC-05797 (SEQ ID NO:28)
DBC-29907 (SEQ ID NO:29)

Both cassettes encoding the same growth factor were integrated at the pTEFUP1 locus of the *Pichia pastoris* host cell, on Chromosome I as described in (Liu, Q., Shi, X., Song, L., Liu, H., Zhou, X., Wang, Q., et al. (2019). CRISPR-Cas9-mediated genomic multiloci integration in *Pichia pastoris.* Microbial Cell Factories, 18(1).) The pCASPP backbone was PCR amplified from pCASPP-lig1 using primers DBC-26957 (SEQ ID NO:4) and DBC-26958 (SEQ ID NO:5) using Phusion polymerase. The gRNA's were PCR amplified from the ordered gBlocks using DBC-26287 (SEQ ID NO:6) and DBC-26288 (SEQ ID NO:7) using Phusion polymerase. A Gibson reaction was performed to assembly the gRNA into the pCASPP backbone to yield a functional AIO plasmid.

Competent PPS02 was transformed with 100 ng plasmid pCASPP-01 and 500 ng of the A-B and B_C expression cassettes. Plating was done on 48 well Q-trays containing Selective G418 (750 ug/ml) yepD agar. Ten times and a fifty times dilution were plated and incubation at 30 °C for 3 days.

Colonies were checked by diagnostic PCR, for correct 5' integration with primers
DBC-27547 (SEQ ID NO:30) ACTTGACCCGTCATACAGGC
DBC-05794 (SEQ ID NO:31) AAAGCAAAGGAAGGAGAGAAC

Colonies were checked by diagnostic PCR for correct 3' integration with primers
DBC-05799 (SEQ ID NO:32) ACGCTTTCCGGCATCTTCCAG
DBC-27548 (SEQ ID NO:33) GCCAATCATGTACGAGTGCG

The obtained strains expressing the growth factors, are listed in table 1.

**Table 1**

| Strain name | Variant name | Description variant | SEQ ID NO: of the polypeptide | SEQ ID NO: of polynucleotide encoding the polypeptide |
|---|---|---|---|---|
| PP301 | FGF301 | FGF2_native_Pig | 11 | 16 |
| PP320 | FGF320 | FGF1+FGF2-CHIMERA_Pig | 12 | 17 |
| PP321 | FGF321 | FGF1+FGF2-CHIMERA_Pig_ C12S; C113A | 13 | 18 |
| PP322 | FGF322 | FGF1+FGF2_CHIMERA_Pig_ Q43I; A62C; H89G; K108T; | 14 | 19 |
| PP323 | FGF323 | FGF1+FGF2-CHIMERA_Pig_ C12S; Q43I; A62C; H89G; C113A | 15 | 20 |

### Production of recombinant Growth Factors in P. pastoris

From the Q-tray agar plates transformants were inoculated into 200 µl YephD medium (10 g/l Yeast Extract, 20 g/l Phytone peptone and 20 g/l glucose) in HDWP using a pin and incubated overnight in an INFORS MTP incubator at 30°C, 750 rpm and 80% humidity. These cultures were used as pre-culture for the protein production. From the 200 µl pre-culture, 20 µl was inoculated into 2 ml YephD per well at 28 degrees centigrade, 320 rpm, 90 percent humidity in a 24-deepwell plate covered with a breathable seal. Incubation of the plate was done in an INFORS MTP incubator at 28°C, 550 rpm and 80% humidity for 3 days. Intracellular samples were harvested by filling a Low Binding Protein Plate with 750 ul broth. The plates were centrifuged at 2750 g for 10 minutes. The supernatants were discarded by gently inverting the plate and dried on tissues. The cell pellets (from 750 ul broth) were resuspended in 0.5 ml lysis buffer (0,2 M Tris-HCI buffer with 0,1 M NaCl and 50 mM L-cysteine pH 8.3 set by 0,4 N HCL) to which 5 µL octanol was added. This was incubated at RT for 3 hours in a thermomixer (400 rpm). The samples were centrifuged and the supernatants were stored at -20 °C until further activity analysis. Intracellular production of the growth factors was confirmed by LC-MS and activity is determined as below.

### LC-MS analysis intact protein

Standards recombinant (*E*. *coli*) human FGF2 (Peprotech 100-18B) and cation exchange purified supernatants were analyzed with a Synapt G2-s (Waters) equipped with a Acquity I-Class LC (Waters). The chromatographic system consisted of a BEH C4 300A 1.7um 2.1*50 column (Waters), which was kept at 75°C and gradient elution using A: 0.1 % Formic Acid in water, and B: Formic Acid 0,1% in Acetonitrile : Water 90 : 10 (v/v), and a flow-rate of 400 ul/min. The gradient started at 3% B for 2 minutes, then linear increasing to 17% B in 0.2 minutes, following by linear increasing to 66% B in 5.8 minutes, directly increasing to 95% and kept here for 1 minutes, then directly decreasing to 3% B and kept here for 5 minutes, for re-equilibrating the LC-MS system. Standard, recombinant (*E. coli*) human FGF2 used to test the intact protein LC-MS system. In Electrospray ionization mass spectrometry (ESI-MS), proteins are characterized by multiplycharged species.

With deconvolution software (Waters Maxent1) the average mass could be calculated. For FGF2 (Mavg = 17122.5) standard the correct average mass of the amino acid sequence was obtained.

### Activity measurement by HEK293 reporter cell line

The supernatants of the lysed samples from the HDWP produced FGF variants were used to analyze their stability. From each FGF variant, 400 µL of supernatant was incubated at 37°C in a thermomixer (Eppendorf) at 600 rpm after which samples of 50 µL were taken after 0, 3, 6, 24 and 48 hours. Samples were immediately frozen and stored at -80°C until their activity was determined. Activity in the SRE Reporter - HEK293 Cell Line (ERK Pathway) (AMSBIO) was determined according to the supplier's protocol. The cells were grown in a T75 flask using Growth medium (BPS Bioscience, 79531) and incubated at 37°C at 5% CO2 (New Brunswick S41i, Eppendorf). When cells are about 80% confluent, medium was removed, cells were washed 1 x with 10 mL PBS after which 1 mL 0.05% Trypsin/0.02% EDTA (Sigma-Aldrich, 59417C) was added to detach the cells. After 5 minutes at RT 6 mL growth medium was added, cells were counted (Nucleocounter NC-200, ChemoMetec) and the cells were spun down using a centrifuge for 5 min at 200 rcf at RT. The pellet was resuspended to 300.000 cells/ml in fresh Growth medium after which 100 µl was added to each well of a Cellstar flat bottom 96 well MTP (Greiner, 655180). The MTP plate was incubated at 37°C at 5% CO2 for 24 h after which the growth medium was removed and 90 µL Assay medium (BPS Bioscience, 79617) was added. The MTP plate was incubated again for 24h at 37°C at 5% CO2.

The FGF samples were diluted to 4000 ng FGF/mL in sterile PBS pH 7.4 (Gibco, 10010023). From this a serial dilution (factor 3 each step) was made in the Assay medium to get a concentration range from 4000 - 0.07 ng FGF/mL. From each sample dilution, 10 µl was added to the MTP plate containing the HEK293 cells. For 8 wells only 10 µl of assay medium was added (white wells). The MTP plate was then incubated at 37°C at 5% CO2 for 6h. Subsequently, 60 µl of the medium was removed from each well and 40 µl ONE-Step^{™} Luciferase Assay System (BPS Bioscience, 60690) was added after which the luminescence was measured on a MTP luminometer (Tecan SPECTRA Fluor Plus Microplate Reader). The dose-response curve was obtained from which an EC50 value, the FGF concentration that results in 50% of the maximum luminescence of that sample (1/2 max, response), was calculated as follows; the average luminescence of the Blancs were subtracted from the luminescence of all sample dilutions. For each sample the maximum luminescence response was taken and divided by 2 to get the ½ max. response. Next a linear regression line (y=ax+b) was fitted through the linear part of the dose-response data to obtain the slope and the intercept values. Based on the ½ max. response, the accompanying FGF concentration can be calculated referred to as EC50 value (Table 2). In the table, a lower EC50 value reflects a higher concentration of active FGF.
Y = luminescence
a= slope
x= FGF concentration (ng/mL)
b= intercept

**Table 2: EC50 values measured for the FGF samples in the HEK293 activity assay**

| | EC50 (ng FGF/mL) | | |
|---|---|---|---|
| Incubation time at 37°C (h) | FGF301 | FGF320 | FGF323 |
| 0 | 0.4 | 16 | **0.3** |
| 3 | 1.6 | 37 | **0.7** |
| 6 | 0.9 | >100 | **0.4** |
| 24 | 12.5 | >400 | **0.7** |
| 48 | 26.8 | >400 | **1.5** |

### Activity measurement by ELISA

The supernatants of the lysed samples from the HDWP produced FGF variants were used to analyze their stability. From each FGF variant, 400 µL of supernatant was incubated at 37°C in a thermomixer (Eppendorf) at 600 rpm after which samples of 50 µL were taken after 0, 3, 6, 24 and 48 hours. Samples were immediately frozen and stored at -80°C until their activity was determined.

A FGF receptor (Recombinant Human FGFR1 alpha (IIIc) Fc Chimera Protein) (R&D systems, 658-FR-050) stock solution was made at 0.5 µg/ml D-PBS buffer (Sigma, D1408-500ML). From this stock solution 100 µL was added to each MTP well in a 96-well plate (Clear flat bottom immuno nonsterile 96 well plates (Maxisorp hydrophilic), Thermo scientific, 442404), which was incubated for 16h at 23°C and 300 rpm. Subsequently, the MTP plate was washed 3x with 250 µL D-PBS containing 0.05% Tween after which 200 µL D-PBS including 1% Naturalys S85M pea protein (Roquette, W707P) was added to each well. The MTP plate was incubated for 2h at 23°C and 300 rpm and subsequently washed 3× with 250 µL D-PBS containing 0.05% Tween. Next 100 µL of the FGF samples with a concentration of 80 ng/mL diluted in D-PBS, were added to at least 3 different wells for a triplicate measurement. To at least 3 wells only D-PBS buffer was added, referred to as blank. The MTP was incubated for 2h at 23°C and 300 rpm followed by washing 3x with 250 µL D-PBS including 0.05% Tween. Next 100 µL of 1 µg/ml FGF biotinylated antibody (R&D systems, BAM233) diluted in D-PBS was added to each well. The MTP plate was incubated for 2h at 23°C and 300 rpm followed by washing 3x with 250 µL D-PBS including 0.05% Tween. Then 100 µL of a Streptavidin Horseradish Peroxidase solution (400x diluted in D-PBS) (Tocris, RABHRP3-600UL) was added to each well followed by an incubation for 40 min at 23°C and 300 rpm. The MTP plate was washed 3x with 250 µL D-PBS including 0.05% Tween after which 100 µL of 3,3',5,5' tetramethylbenzidine (TMB) solution including H₂O₂ (Sigma, T0440-1L) was added to each well and the plate was incubated for 30 min at RT under dark conditions. Then 50 µL of 1M H₂SO₄ was added to each well and absorbance was directly measured at 450 nm (Multiskan SkyHigh Microplate UV/Vis Spectrophotometer, Thermo Scientific).

The average absorbance measured at 450 nm for the wells were only D-PBS buffer was added (blank) was subtracted from the absorbance measured for the FGF samples (Table 3).
FGF323 remains active even after 48 hours incubation at 37 C. This is illustrated by the high absorbance > 2. The activity of FGF301 and FGF320 drops which is demonstrated by the lower absorbance (450 nM) below 2 and below 1 due to the longer incubation ay 37 C.

**Table 3: Absorbance (450 nm) measured for the FGF samples in the ELISA activity assay**

| Absorbance | Absorbance (450 nm) | | |
|---|---|---|---|
| Incubation time at 37°C (h) | FGF301 | FGF320 | FGF323 |
| 0 | 2.3 | 2.1 | **2.5** |
| 6 | 2.1 | 0.9 | **2.4** |
| 24 | 1.5 | 0.7 | **2.3** |
| 48 | 0.8 | 0.6 | **2.3** |

## Claims

1. An isolated polypeptide, or a functional fragment thereof, having at least 85% sequence identity to SEQ ID NO: 12, comprising an amino acid substitution at a position selected from the group consisting of: C12, Q43, A62, H89 and C113.

2. An isolated polypeptide, or a functional fragment thereof, according to claim 1, wherein the amino acid substitutions are selected from the group consisting of C12S, Q43I, A62C, H89G and C113A.

3. An isolated polypeptide, or a functional fragment thereof, according to any one of the preceding claims, wherein the polypeptide comprises or consists of polypeptide with the sequence as set forward in SEQ ID NO's 13 to 15.

4. An isolated polypeptide, or a functional fragment thereof, according to any one of the preceding claims, having a residual activity of at least 50%, at least 60%, at least 70%, at least 80%, at least 90%, at least 95 or at least 99% of its activity at t=0, after 24 hours or 48 hours incubation at 37°C, preferably in a standard buffer.

5. A polynucleotide encoding a polypeptide according to anyone of claims 1 to 4.

6. An expression vector comprising the polynucleotide according to claim 5.

7. A host cell comprising the expression vector according to claim 6.

8. A method for the production of the polypeptide according to anyone of claims 1 to 4, comprising culturing the host cell according to claim 7 under conditions conducive to the production of the polypeptide, and optionally, isolating and/or purifying the polypeptide from the broth.

9. A culture medium comprising the polypeptide according to any one of claims 1 to 4.

10. A method for culturing cells comprising incubating cells in a culture medium comprising the polypeptide according to any one of claims 1 to 4, wherein the cells preferably are non-human mammalian cells.

11. A cultured cell obtainable by the method according to claim 10.

12. A method for the production of a food product comprising culturing non-human mammalian cells in a culture medium comprising the polypeptide according to any one of claims 1 to 4, and optionally, isolating and/or purifying the food product from the broth.

13. A food product obtainable by the method according to claim 12.
